**Europäisches Patentamt**

**European Patent Office**

**Office européen des brevets**

(19)

(11) Publication number : **0 686 403 A1**

(12) # EUROPEAN PATENT APPLICATION

(21) Application number : **95108828.5**

(22) Date of filing : **08.06.95**

(51) Int. Cl.[6] : **A61M 1/02**

(30) Priority : **09.06.94 JP 152635/94**

(43) Date of publication of application :
**13.12.95 Bulletin 95/50**

(84) Designated Contracting States :
**DE FR IT NL SE**

(71) Applicant : **TERUMO KABUSHIKI KAISHA**
No. 44-1, Hatagaya 2-chome,
Shibuya-ku
Tokyo 151 (JP)

(72) Inventor : **Tanokura, Nobukazu, c/o Terumo Kabushiki Kaisha**
818, Misonodaira
Fujinomiya-shi, Shizuoka-ken (JP)
Inventor : **Ishida, Noboru, c/o Terumo Kabushiki Kaisha**
818, Misonodaira
Fujinomiya-shi, Shizuoka-ken (JP)
Inventor : **Hosono, Norio, c/o Terumo Kabushiki Kaisha**
818, Misonodaira
Fujinomiya-shi, Shizuoka-ken (JP)

(74) Representative : **Casalonga, Axel et al**
BUREAU D.A. CASALONGA - JOSSE
Morassistrasse 8
D-80469 München (DE)

(54) **Multiple bag and method for separation and transfer of blood components**

(57)  A multiple bag 1 is a quadruple bag having a blood collecting bag 10, a blood plasma bag 20, a buffy coat bag 30, and a chemical liquid storing bag 40 interconnected severally with prescribed tubes. The blood collecting bag 10 and the buffy coat bag 30 are connected with a tube 16 to be used exclusively for transferring buffy coat obtained by separating the blood in the blood collecting bag 10. In the neighborhood of the part for connecting the tube 16 to the buffy coat 30, a sealing member 34 capable of being broken so as to open an inner flow path is provided. When the inner flow path inside the sealing member 34 is opened, the blood collecting bag 10 and the buffy coat bag 30 are allowed to communicate with each other through the medium of the tube 16 and the sealing member 34. This multiple bag 1 permits improvement of the ratio of recovery or ratio of removal of blood components, particularly the ratio of removal of leukocyte, without requiring use of any bag of special construction.

FIG. I

EP 0 686 403 A1

This invention relates to a multiple bag formed by interconnecting through the medium of tubes a plurality of bags for receiving and storing therein blood components separated from blood and a method for the separation and transfer of blood components by the use thereof.

In the blood transfusion, the system of separating the blood extracted from a donor into components by means of centrifugal separation, for example, and using only the components necessary for a recipient is prevailing today by such reasons as ensuring effective utility of blood and alleviating the burden on the part of blood donors. This transfusion of blood components is now promising effective utility of blood as compared with the conventional transfusion of whole blood.

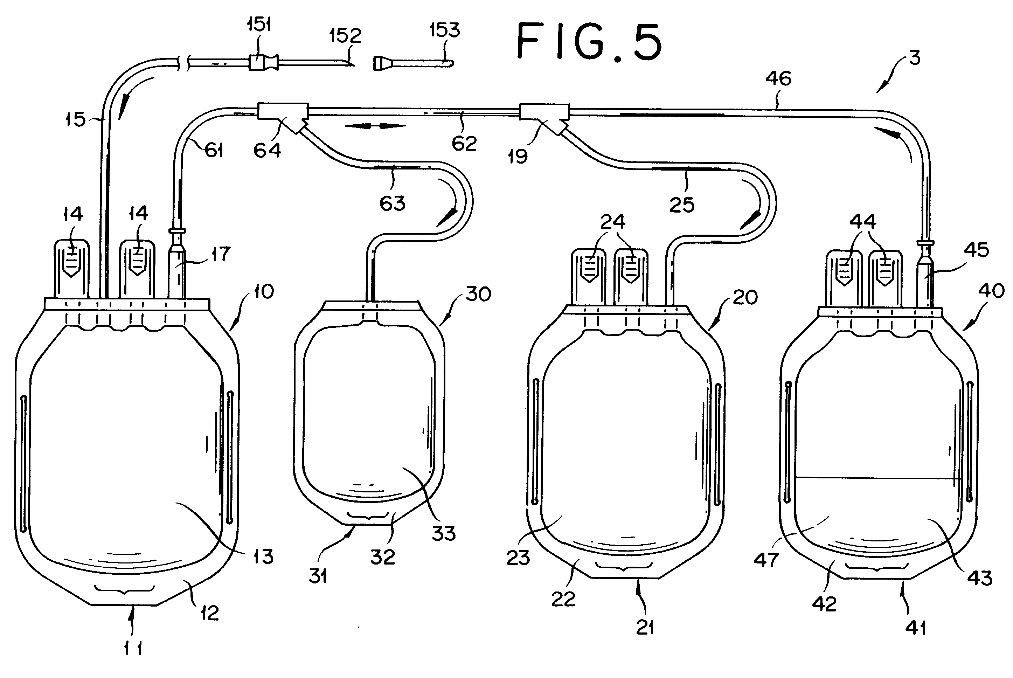

For the transfusion of components, a multiple bag which is formed by interconnecting with tubes a plurality of bags including blood collecting bags is used. The construction of a quadruple bag, one of the various forms of the multiple bag, is shown in Fig. 5.

A quadruple bag 3, as shown in Fig. 5, has a blood collecting bag 10, a blood plasma bag 20, a buffy coat bag 30, and a chemical storage bag (platelet bag) 40 interconnected with tubes. In this case, the blood collecting bag 10 is provided in the neighborhood of the upper outlet thereof with a sealing member 17 which, on being broken, opens an inner flow path.

In the quadruple bag 3, the blood collected in the blood collecting bag 10 is separated by centrifugation into three layers formed respectively of erythrocyte, buffy coat, and blood plasma. Then, the sealing member 17 is opened to transfer the blood plasma of the upper layer via tubes 61, 62, and 25 into the blood plasma bag 20 and the buffy coat of the intermediate layer via the tube 61 and a tube 63 into the buffy coat bag 30, with the erythrocyte of the lower layer left behind in the blood collecting bag 10. Subsequently, an erythrocyte conserving liquid 47 in the chemical liquid storage bag 40 is transferred via a tube 46 and the tubes 62 and 61 into the blood collecting bag 10. Thereafter, the midway part of the tube 25 is fused and cut to separate the blood collecting bag 10 and the blood plasma bag 20. Then, the buffy coat in the buffy coat bag 30 is separated by centrifugation into a platelet layer and a leukocyte layer and the platelets floating on the upper layer are transferred into the chemical storage bag 40 which has been emptied.

In the quadruple bag 3, however, during the transfer of the buffy coat to the buffy coat bag 30, the leukocyte in the buffy coat stagnate and accumulate near the broken segment remaining inside the sealing member 17 and prevent the transfer from proceeding smoothly. Further, during the transfer of the erythrocyte preserving liquid 47 to the blood collecting bag 10, since the erythrocyte preserving liquid 47 passes through the interior of the sealing member 17 and pushes the accumulated leukocyte back into the blood collecting bag 10, the ratio of removal of leukocyte from within the erythrocyte remaining inside the blood collecting bag 10 is lowered.

Incidentally, as a blood collecting bag of special construction, the blood collecting bag (top and bottom bag) which has an upper tube for discharging blood plasma connected to the upper end and a lower tube for discharging erythrocyte connected to the lower end thereof has been known (JP-B-63-20,144). The multiple bag incorporating this blood collecting bag therein is free from the problem remarked above because it is so constructed as to have the remaining free ends of the upper and the lower tube mentioned above connected respectively to the blood plasma bag and the erythrocyte storing bag (hereinafter referred to as "erythrocyte bag") and, therefore, enable the blood collected in the blood collecting bag to be separated by centrifugation into three layers of erythrocyte, buffy coat, and blood plasma, permit the erythrocyte of the lower layer and the blood plasma of the upper layer to be transferred respectively to the erythrocyte bag and the blood plasma bag via the lower and the upper tube, and allow the buffy coat of the intermediate layer to remain undischarged from the blood collecting bag.

This multiple bag, however, is at a disadvantage in requiring a device (such as, for example, a product of Baxter Corp. marketed under trademark designation of "Optipress") to be exclusively used for simultaneously discharging the blood plasma and the erythrocyte resulting from the separation respectively through the upper and the lower part of the blood collecting bag and, moreover, consuming a long time for the purpose of discharging highly viscous erythrocyte and transferring them to the erythrocyte bag. Further, this multiple bag is not adapted for the work of centrifugation on account of construction. To be specific, since the blood collecting bag has the tubes severally connected to the upper and the lower end thereof, this bag permits no easy discrimination between the upper and the lower side thereof prior to the work of centrifugation, impairs the ease with which the component bags of the multiple bag are bundled during their insertion into a centrifugation cup, gives rise to a dead space in the bottom part of the centrifugation cup, and tends to sustain damage and breakage.

An object of this invention is to provide a multiple bag which is capable of exalting the ratio of recovery and the ratio of removal of blood components without requiring use of bags of special construction and a method for the separation and the transfer of blood components.

This object is accomplished by the following aspects of this invention set forth in (1) through (11) below.

(1) A multiple bag comprising a first bag for storing blood, a second bag for storing a first blood component

EP 0 686 403 A1

formed mainly of blood plasma separated from the blood mentioned above, a third bag for storing a second blood component containing leukocyte separated from the blood mentioned above, a first tube for interconnecting the first bag and the second bag in such a manner as to establish communication between the interiors of the bags, and a second tube for interconnecting the first bag and the third bag in such a manner as to establish communication between the interiors of the bags, characterized in that a sealing member capable of being broken so as to open an inner flow path is provided in the neighborhood of the part for connecting the second tube to the third bag and, by opening the inner flow path of the sealing member, the first bag and the third bag are enabled to communicate with each other through the medium of the second tube.

(2) A multiple bag set forth in (1) above, wherein the sealing member mentioned above is so constructed that a broken fragment separated therefrom by breakage is drawn in to a position at which it is incapable of interfering with the flow of the blood component therethrough.

(3) A multiple bag set forth in (1) or (2) above, wherein the first bag mentioned above is used as a bag for receiving and storing a third blood component containing erythrocyte separated from the blood.

(4) A multiple bag set forth in any of (1) through (3) above, which further comprises a fourth bag containing a chemical liquid.

(5) A multiple bag set forth in (4) above, which further comprises a third tube branching from the first tube mentioned above and connected to the fourth bag mentioned above so as to communicate with the interior of the fourth bag mentioned above.

(6) A multiple bag set forth in (4) above, which further comprises a third tube branching from the second tube mentioned above and connected to the fourth bag mentioned above so as to communicate with the interior of the fourth bag mentioned above.

(7) A multiple bag set forth in (6) above, wherein the fourth bag mentioned above is used as a bag for receiving and storing a fourth blood component containing platelets and separated further from the second blood component mentioned above.

(8) A method for separating and transferring blood components by the use of a multiple bag set forth in any of (1) through (7) above, characterized by comprising a step of subjecting the blood stored in the first bag mentioned above to centrifugation thereby separating the blood into at least three components, i.e. an upper layer formed of the first blood component mentioned above, an intermediate layer formed of the second blood component mentioned above, and a lower layer formed of the third blood component containing erythrocyte, a step of transferring the first blood component mentioned above to the second bag mentioned above through the medium of the first tube mentioned above, and a step of transferring the second blood component mentioned above to the third bag mentioned above through the medium of the second tube mentioned above and the sealing member mentioned above in an opened state.

(9) A method for separating and transferring blood components by the use of a multiple bag set forth in (5) above, characterized by comprising a step of subjecting the blood stored in the first bag mentioned above to centrifugation thereby separating the blood into at least three components, i.e. an upper layer formed of the first blood component mentioned above, an intermediate layer formed of the second blood component mentioned above, and a lower layer formed of the third blood component containing erythrocyte, a step of transferring the first blood component mentioned above to the second bag mentioned above through the medium of the first tube mentioned above, a step of transferring the second blood component mentioned above to the third bag mentioned above through the medium of the second tube mentioned above and the sealing member mentioned above in an opened state, and a step of transferring the chemical liquid in the fourth bag mentioned above to the first bag mentioned above through the medium of the third tube mentioned above and the first tube mentioned above.

(10) A method for separating and transferring blood components by the use of a multiple bag set forth in (6) or (7) above, characterized by comprising a step of subjecting the blood stored in the first bag mentioned above to centrifugation thereby separating the blood into at least three components, i.e. an upper layer formed of the first blood component mentioned above, an intermediate layer formed of the second blood component mentioned above, and a lower layer formed of the third blood component containing erythrocyte, a step of transferring the first blood component mentioned above to the second bag mentioned above through the medium of the first tube mentioned above, a step of transferring the second blood component mentioned above to the third bag mentioned above through the medium of the second tube mentioned above and the sealing member mentioned above in an opened state, and a step of transferring the chemical liquid in the fourth bag mentioned above to the first bag mentioned above through the medium of the third tube mentioned above and the second tube mentioned above.

(11) A method for separating and transferring blood components by the use of a multiple bag set forth in (6) or (7) above, characterized by comprising a step of subjecting the blood stored in the first bag mentioned above to centrifugation thereby separating the blood into at least three components, i.e. an upper layer formed of the first blood component mentioned above, an intermediate layer formed of the second blood component mentioned above, and a lower layer formed of the third blood component containing erythrocyte, a step of transferring the first blood component mentioned above to the second bag mentioned above through the medium of the first tube mentioned above, a step of transferring the second blood component mentioned above to the third bag mentioned above through the medium of the second tube mentioned above and the sealing member mentioned above in an opened state, a step of transferring the chemical liquid in the fourth bag mentioned above to the first bag mentioned above through the medium of the third tube mentioned above and the second tube mentioned above, a step of subjecting the second blood component mentioned above stored in the third bag mentioned above thereby obtaining an upper layer mainly containing platelets, and a step of transferring the platelets of the upper layer mentioned above to the fourth bag mentioned above through the medium of the second tube mentioned above and the third tube mentioned above.

According to the multiple bag of this invention and the method for the separation and transfer of blood components by the use of the multiple bag, the separation of blood into a plurality of blood components and the transfer of the separated blood components to and the recovery thereof in the component bags of the multiple bag can be carried out sterilely and quickly as described above. Since the sealing member is disposed not in the neighborhood of the outlet for discharging the second blood component from the first bag but in the neighborhood of the inlet to the third bag, the transfer of the second blood component to the third bag is effected smoothly and, as a consequence, the necessity for using such a blood collecting bag of special construction as a bottom and top bag having tubes severally connected to the upper and the lower side thereof is obviated and the ratio of recovery and the ratio of removal of blood components are as high as or higher than the conventional multiple bag.

Particularly when this invention is used for the manufacture of erythrocyte products, the removal of leukocyte (especially lymphocytes) from blood is attained at a heightened ratio. The use of the erythrocyte products consequently obtained, therefore, precludes the occurrence of unhemolytic reactions.

When the sealing member to be used is so constructed that, when it is broken, a fragment consequently separated therefrom is drawn back to a position at which it is not suffered to obstruct the flow of a blood component passing therethrough, the introduction of the second blood component into the third bag and the discharge of platelets in the second blood component from the third bag are implemented more smoothly, the advantageous effects mentioned above are manifested more conspicuously, and the time consumed for the separation of blood components is shortened.

Fig. 1 is a plan view showing an example of the construction of a multiple bag of this invention.

Fig. 2 is a plan view showing another example of the construction of a multiple bag of this invention.

Fig. 3 is a longitudinal cross section showing as magnified an example of the construction of a sealing member used in this invention.

Fig. 4 is a longitudinal cross section showing as magnified another example of the construction of a sealing member used in this invention.

Fig. 5 is a plan view showing an example of the construction of a conventional multiple bag.

Now, the multiple bag of this invention and the method for the separation and transfer of blood components will be described in detail below with reference to the embodiments illustrated in the annexed drawings.

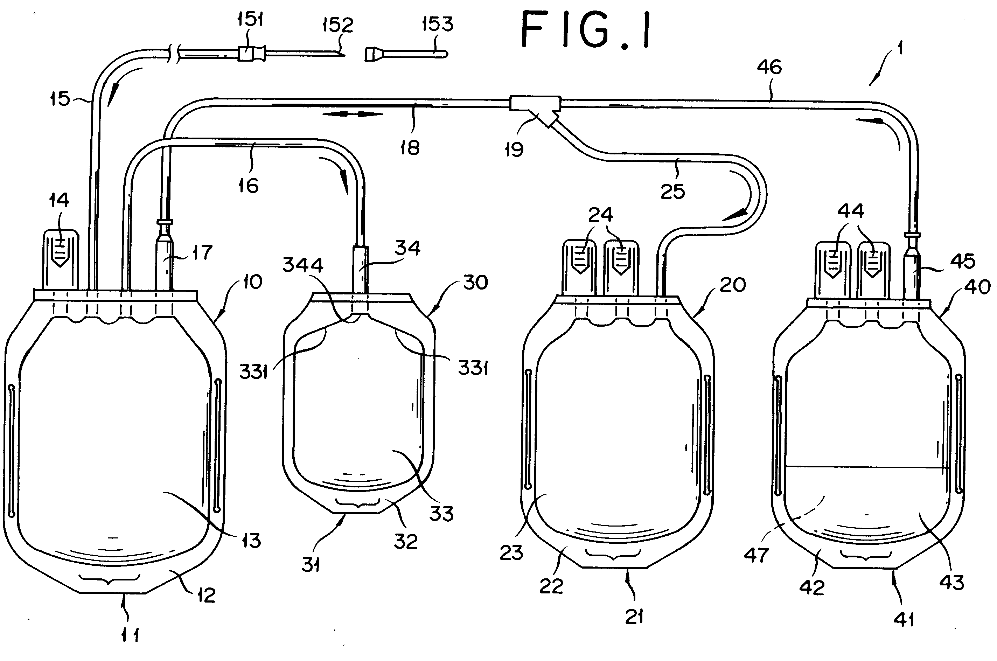

Fig. 1 is a plan view showing an example of the construction of a multiple bag of this invention. As shown in this diagram, a multiple bag 1 is a quadruple bag having a blood collecting bag (first bag) 10, a blood plasma bag (second bag) 20, a buffy coat bag (third bag) 30, and a chemical liquid storing bag (fourth bag) 40 interconnected with prescribed tubes.

The blood collecting bag 10 comprises a bag body 11 which is constructed in the shape of a bag by superposing flexible sheets of resin which will be more specifically described hereinbelow and melting (thermal melting, high frequency melting, etc.) or welding the superposed resin sheets in a seal part 12 along the matched edges of sheets.

A blood storing part 13 for storing the collected blood is formed in the inner part of this bag body 11 which is enclosed with the seal part 12. Further, since the blood collecting bag 10 is concurrently used as an erythrocyte bag for storing erythrocyte (third blood component), the blood storing part 13 eventually receives and stores concentrated erythrocyte.

The bag body 11 is provided in the upper part thereof with an outlet 14 openably sealed with a peel tab and a sealing member 17. Further, flexible tubes 15 and 16 are each connected at one end thereof to the upper part mentioned above. The interiors of the outlet 14, the tubes 15 and 16, and a sealing member 17 invariably communicate with the blood storing part 13.

To the remaining end of the tube 15 is attached a blood collecting needle 152 through the medium of a hub 151. Further, a cap 153 for wrapping the blood collecting needle 152 is attached to the hub 151. A blood collecting line is composed of the tube 15, hub 151, blood collecting needle 152, and cap 153.

One end of a flexible tube 18 is connected to the sealing member 17 and the other end of the tube 18 is connected to one end of the tube 25 through the medium of a branching connector 19 having the shape of the letter T with a slanted downstroke. A first tube is composed of the tubes 18 and 25. The sealing member 17 is so constructed that the inner flow path thereof is in a closed state before the sealing member 17 is broken and the inner flow path assumes an opened state when the sealing member 17 is broken. Examples of the construction of this sealing member 17 will be described more specifically afterward.

The blood plasma bag 20 comprises a bag body 21 which is constructed in the shape of a bag by superposing flexible sheets of resin which will be more specifically described hereinbelow and melting (thermal melting, high frequency melting, etc.) or welding the superposed resin sheets in a seal part 22 along the matched edges of sheets.

A blood plasma storing part 23 for storing the blood plasma (first blood component) separated from the blood in the blood collecting bag 10 is formed in the inside part of the bag body 21 which is enclosed with the seal part 22.

In the upper part of the bag body 21, two outlets 24 openably sealed with a peel tab and adapted for transfusion are formed. Beside the outlets 24, the other end of the tube 25 is connected to the upper part of the bag body 21 in such a manner as to communicate with the blood plasma storing part 23. When the inner flow path of the sealing member 17 is opened, therefore, the blood storing part 13 of the blood collecting bag 10 and the blood plasma storing part 23 of the blood plasma bag 20 are allowed to communicate with each other through the medium of the sealing member 17, the tubes 18 and 25, and the branching connector 19.

The buffy coat bag 30 comprises a bag body 31 which is constructed in the shape of a bag by superposing flexible sheets of resin which will be more specifically described hereinbelow and melting (thermal melting, high frequency melting, etc.) or welding the superposed resin sheets in a seal part 32 along the matched edges of sheets.

A buffy coat storing part 33 for storing the buffy coat (second blood component) separated from the blood in the blood collecting bag 10 is formed in the inner part of this bag body 31 which is enclosed with a seal part 32.

The bag body 30 is provided in the upper central part thereof with a sealing member 34 so constructed that an inner flow path thereof is in a closed state before the sealing member 34 is broken and the inner flow path assumes an opened state when the sealing member 34 is broken. The other end of the tube 16 (second tube) mentioned above is connected to the upper end side of the sealing member 34 and the inner flow path of the sealing member 34 communicates with the buffy coat storing part 33 through the medium of a lower end opening 344 thereof. When the flow path of the sealing member 34 is opened, therefore, the blood storing part 13 of the blood collecting bag 10 and the buffy coat storing part 33 of the buffy coat bag 30 are allowed to communicate with each other through the medium of the sealing member 34 and the tube 16.

The upper part of the buffy coat storing part 33 in the bearings of Fig. 1 is slanted from the lower end opening 344 of the sealing member 34 downward to the left and the right to form diverging shoulders 331. Owing to the shape consequently assumed by the buffy coat storing part 33, during the operation of discharging the buffy coat from the buffy coat storing part 33 and pooling it in a buffy coat pooling bag at such a step as "A-9" which will be specifically described afterward, the buffy coat flows along the two diverging shoulders 331 and into the lower end opening 344 of the sealing member 34. As a result, the pooling of the buffy coat is easily attained and the ratio of recovery of the buffy coat is improved and the ratio of recovery of platelets is exalted at the steps "A-10 to A-12" which will be specifically described afterward.

The chemical liquid storing bag 40 comprises a bag body 41 which is constructed in the shape of a bag by superposing flexible sheets of resin which will be more specifically described hereinbelow and melting (thermal melting, high frequency melting, etc.) or welding the superposed resin sheets in a seal part 42 along the matched edges of sheets.

A chemical liquid storing part 43 for storing a chemical liquid is form in the inside part of the bag body 41 which is enclosed with the seal part 42. In the present example, the chemical liquid storing part 43 stores a prescribed amount of an erythrocyte preserving liquid 47. As typical examples of the erythrocyte preserving liquid, a S.A.G.M. liquid (aqueous solution containing 0.877 W/V % of sodium chloride, 0.0169 W/V % of adenine, 0.818 W/V % of grape sugar, and 0.525 W/V % of D-mannitol), an OPTISOL liquid, an ADSOL liquid, and a MAP liquid may be cited.

In the upper part of the bag body 41, two outlets 44 openably sealed with a peel tab are formed. Beside the outlets 44, a sealing member 45 of the same construction as mentioned above is disposed so as to communicate with the chemical liquid storing part 43. To the sealing member 45 is connected one end of a flexible tube (third tube) 46. The other end of the tube 46 is connected to the end parts of the tubes 18 and 25 through the medium of the branching connector 19. When the flow paths of the sealing members 17 and 45 are opened, therefore, the blood storing part 13 of the blood collecting bag 10, the chemical liquid storing part 43 of the chemical liquid storing bag 40, and the blood plasma storing part 23 of the blood plasma bag 20 are allowed to communicate with one another through the medium of the sealing members 17 and 45, the tubes 18, 25, and 46, and the branching connector 19.

Preferably, the blood collecting bag 10 preparatorily contains an anticoagulant. This anticoagulant is normally in a liquid state. As typical examples of the anticoagulant, An ACD-A liquid, a CPD liquid, a CPDA-1 liquid, and a sodium heparin liquid may be cited. The amount of the anticoagulant so prepared must be proper for the amount of blood to be collected.

Optionally, the buffy coat bag 30 may preparatorily contain a blood platelet preserving liquid. As typical examples of the blood platelet preserving liquid, physiological saline solution, a GAC liquid, a PAS liquid, a PSM-1 liquid, and synthetic storage medium liquid may be cited.

In the multiple bag 1 constructed as described above, since the blood collecting bag 10 has the points of its connection to the tubes 15, 16, 18, etc. concentrated on the upper end side of the bag body 11, the possibility of the blood collecting bag 10 being placed upside down in the centrifugation cup when it is prepared for centrifugation is precluded. Further, the multiple bag 1 enjoys ease of handling because the convenience with which the component bags 10 to 40 of the multiple bag 1 are bundled is great. Moreover, the dead space inevitably produced in the centrifugation cup is decreased and consequently the possibility of the bag being broken by the pressure during the centrifugation is prevented.

When the buffy coat which is held as a separated component in the blood collecting bag 10 is to be transferred to the buffy coat bag 30 through the medium of the tube 16, the possibility of leukocyte stagnating and accumulating in the upper part (the neighborhood of the part for discharging buffy coat) of the blood collecting bag 10 will be nil because the blood collecting bag 10 is not provided at the point of its connection to the tube 16 with any sealing member and the buffy coat bag 30 is provided near the point of its connection to the tube 16 with the sealing member 34. The transfer of the buffy coat can be carried out smoothly and the ratio of removal of leukocyte from the blood components in the blood collecting bag 10 is heightened.

Further, since the tube 16 for transferring the buffy coat to the buffy coat bag 30 is disposed separately from the tubes 46 and 18 for transferring the erythrocyte preserving liquid 47 to the blood collecting bag 10, the possibility of the erythrocyte preserving liquid 47 being contaminated by leukocyte during the addition of the erythrocyte preserving liquid 47 to the erythrocyte in the blood collecting bag 10 is nil. The ratio of removal of leukocyte from the erythrocyte products is further improved.

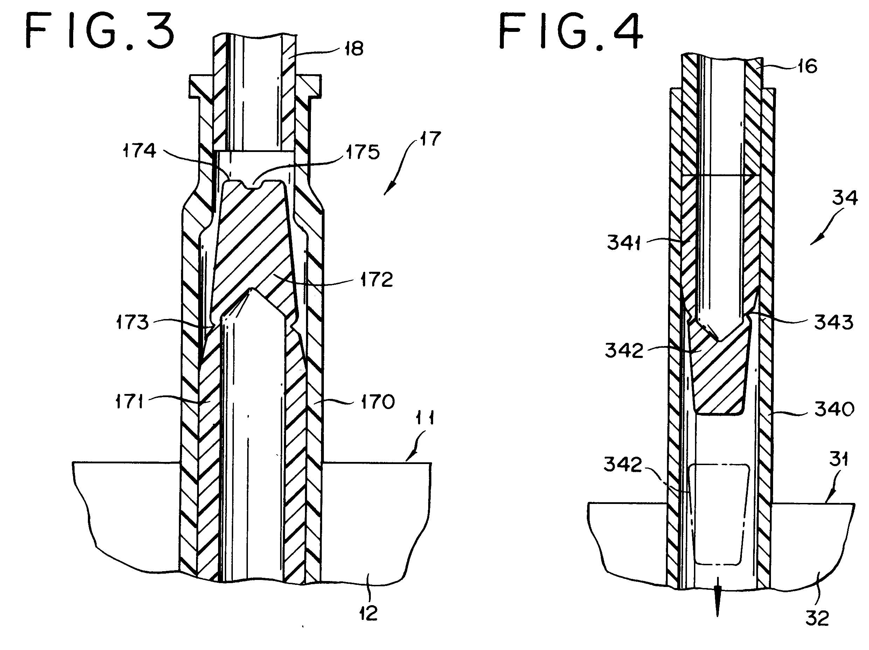

Now, the constructions of the sealing members mentioned above will be described below. Fig. 3 is a longitudinal cross section showing as magnified an example of the construction of the sealing member 17. As shown in this diagram, the sealing member 17 is composed of a short tube 170 formed of a flexible material as described specifically afterward and a tubular member 171 inserted watertightly inside the short tube 170 and having one end thereof closed with a solid columnar part 172.

One end of the tube 18 is watertightly connected to the upper end part of the short tube 170 in the bearings of Fig. 3. The lower end part of the short tube 170 in the bearings of Fig. 3 is watertightly joined or fused to the seal part 12 on the bag body 11 and is allowed to communicate with the blood storing part 13 through the medium of the lower end opening thereof.

A fragile thin-wall breaking part 173 is formed intimately round the tubular member 171. An inner flow path of the sealing member 17 is opened by bending the solid columnar part 172 together with the short tube 170 with the pressure exerted as by finger tips on the outer side of the short tube 170 thereby breaking the breaking part 173 and separating the solid columnar part 172.

The materials which are usable for the construction of the tubular member 171 include such hard materials as hard polyvinyl chloride, polycarbonate, and polyesters, for example.

The upper part of the short tube 170 in the bearings of the diagram (the neighborhood of its part of connection to the tube 18) has the diameter thereof decreased. The upper part of the solid columnar part 172 in the bearings of the diagram is shaped like a wedge. The top part 174 of the solid columnar part 172 is flat and the size thereof in the direction of width (lateral direction in the bearings of the diagram) is smaller than the inside diameter of the part of decreased diameter of the short tube 170 and larger than the inside diameter of the tube 18 so that, after the solid columnar part 172 is broken and separated, the fragment of the solid columnar part 172 avoids clogging the tube 18. Further, as shown in the diagram, a groove 175 for promoting flow of liquid is formed in the top part 174 of the solid columnar part 172.

The sealing member 45 is constructed in the same manner as is shown in Fig. 3.

Fig. 4 is a longitudinal cross section showing as magnified an example of the construction of the sealing member 34. As shown in this diagram, the sealing member 34 is composed of a short tube 340 formed of a flexible material as described specifically afterward and a tubular member 341 watertightly inserted in the short tube 340 and having one end thereof closed with a solid columnar part 342. The materials which are used for the construction of the tubular member 341 are the same as those which are usable for the tubular member 171.

One end of the tube 16 is watertightly connected to the upper end part of the short tube 340 in the bearings of the diagram and the lower end part of the short tube 340 in the bearings of the diagram is watertightly attached or melted to the seal part in the upper part of the bag body 31. In this case, the inside diameter of the short tube 340 is not decreased so that the solid columnar part 342, after being broken, may be moved into and out of the buffy coat storing part 31.

A fragile thin-wall breaking part 343 is formed intimately round the outer periphery of the tubular member 341. An inner flow path of the sealing member 34 is opened by bending the solid columnar part 342 together with the short tube 340 with the pressure exerted as by finger tips on the outer side of the short tube 340 thereby breaking the breaking part 342 and separating the solid columnar part 342.

The solid columnar part 342 which has been broken and separated, as shown by an alternate dash and dot line in Fig. 4, is caused to fall inside the short tube 340 down into the buffy coat storing part 31 of the buffy coat bag 30 by gravitational attraction or by the pressure of a liquid flow supplied from the tube 16. Since the solid columnar part 342 as a fragment is no longer present inside the sealing member 34, the flow of the buffy coat to the buffy coat bag 30 during the process of transfer is not obstructed but allowed to proceed smoothly. As a result, the ratio of removal of leukocyte from the erythrocyte remaining in the blood collecting bag 10 is improved and the time consumed for a series of separations of blood components is shortened.

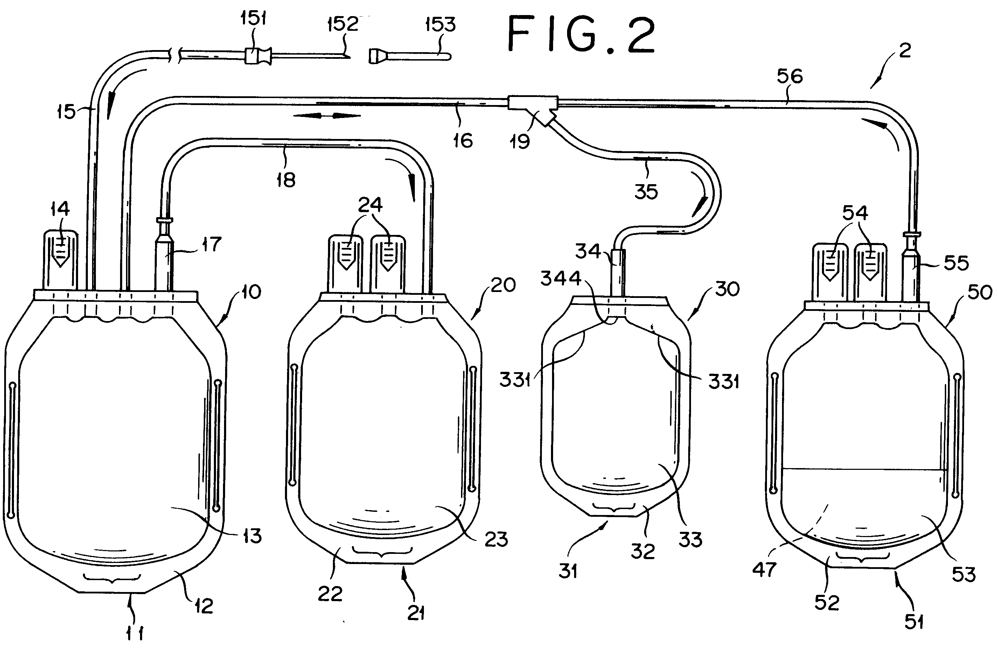

Fig. 2 is a plan view showing another example of the construction of the multiple bag of this invention. As shown in this diagram, a multiple bag 2 is a quadruple bag having a blood collecting bag (first bag) 10, a blood plasma bag (second bag) 20, a buffy coat bag (third bag) 30, and a blood platelet bag (fourth bag) 50 interconnected with prescribed tubes. Now, the parts of the construction of this multiple bag 2 different from the construction of the multiple bag 1 mentioned above will be described and the rest of the construction thereof identical with the construction of the multiple bag 1 will be omitted from the following description.

An outlet 14 and a sealing member 17 are formed in the upper part of a bag body 11 of the blood collecting bag 10 and flexible tubes 15 and 16 are each connected at one end thereof to the upper part. The outlet 14, tubes 15 and 16, and sealing member 17 invariably communicate with a blood storing part 13. The other end of the tube 16 is connected to one end of a tube 35 through the medium of a branching connector 19 having the shape of the letter T with a slanted downstroke. A second tube is formed by these tubes 16 and 35.

To the sealing member 17 is connected one end of a flexible tube (first tube) 18. The other end of the tube 18 is connected to the upper part of the blood plasma bag 20 so as to communicate with a blood plasma storing part 23. When the flow path in the sealing member 17 is opened, therefore, the blood storing part 13 of the blood collecting bag 10 and the blood plasma storing part 23 of the blood plasma bag 20 are allowed to communicate with each other through the medium of the sealing member 17 and a tube 18.

In the upper central part of a bag body 31 of the buffy coat bag 30, a sealing member 34 is disposed. The other end of the tube 35 is connected to the sealing member 34. When the flow path in the sealing member 34 is opened, therefore, the blood storing part 13 of the blood collecting bag 10 and the buffy coat storing part 33 of the buffy coat bag 30 are allowed to communicate with each other through the medium of the tubes 16 and 35, the branching connector 19, and the sealing member 34.

Diverging shoulders 331 are formed in the same manner as described above in the upper part of the buffy coat storing part 33. At a step "B-12" which will be specifically described afterward, therefore, the discharge and transfer of blood platelets can be smoothly carried out. Thus, the yield of platelets is improved.

The blood platelet bag 50 is a bag for receiving and storing blood platelets which are obtained by subjecting the buffy coat stored in the buffy coat bag 30 to centrifugation. It is provided with a bag body 51 which is constructed in the shape of a bag by superposing flexible sheets of resin which will be more specifically described hereinbelow and melting (thermal melting, high frequency melting, etc.) or welding the superposed resin sheets in a seal part 52 along the matched edges of sheets.

A platelet storing part 53 for finally storing blood platelets is formed in the inner part of the bag body 51 which is enclosed with the seal part 52. In the present example, the platelet storing part 53 in the unused state thereof contains a prescribed amount of the erythrocyte preserving liquid 47 mentioned above.

In the upper part of the bag body 51, two outlets 54 openably sealed with a peel tab and adapted for transfusion are formed. Beside the outlets 54, a sealing member 55 of the same construction as mentioned above is disposed so as to communicate with the blood platelet storing part 53. To the sealing member 55 is connected one end of a flexible tube (third tube) 56. The other end of the tube 56 is connected to the tubes 16 and 35 through the medium of the branching connector 19. When the flow paths of the sealing members 34 and 55 are opened, therefore, the blood storing part 13 of the blood collecting bag 10, the blood platelet storing part 53 of the blood platelet bag 50, and the buffy coat storing part 33 of the blood plasma bag 30 are allowed to communicate with one another through the medium of the sealing members 34 and 55, the tubes 16, 35, and 56, and the branching connector 19.

In this multiple bag 2 as in the multiple bag 1 described above, when the buffy coat is to be transferred to the buffy coat bag 30, the possibility of leukocyte stagnating and accumulating in the upper part (the neighborhood of the part for discharging buffy coat) of the blood collecting bag 10 will be nil because the blood collecting bag 10 is not provided at the point of its connection to the tube 16 with any sealing member and the buffy coat bag 30 is provided near the point of its connection to the tube 35 with the sealing member 34. The transfer of the buffy coat can be carried out smoothly. Further, when the erythrocyte preserving liquid 47 is transferred into the blood collecting bag 10 and the erythrocyte preserving liquid 47 is consequently passed in the reverse direction inside the tube 16 which has been used for transferring the buffy coat, the possibility of the leukocyte being returned into the blood collecting bag 10 as entrained by the transfer of the erythrocyte preserving liquid 47 is nil because the tube 16 is not provided with a sealing member which forms a cause for accumulation of leukocyte. As a result, the ratio of removal of leukocyte is improved in the blood components which remain inside the blood collecting bag 10.

Further, in the sealing member 34, the yield of blood platelets is improved because the solid columnar part 342 after being broken is drawn back and, as a result, the introduction of buffy coat to the buffy coat bag 30 and the discharge of blood platelets can be carried out smoothly.

In the multiple bags 1 and 2 shown in Fig. 1 and Fig. 2, flexible polyvinyl chloride is ideally used as the material for the sheet which forms the bag bodies for the bags 10, 20, 30, 40, and 50.

As typical examples of the plasticizer for this flexible polyvinyl chloride, phthalic esters such as di-(2-ethylhexyl) phthalate (DOP) and di-(n-decyl) phthalate (DnDP), adipic esters such as d-(2-ethylhexyl) adipate (DOA), and trimellitic esters such as tri-(2-ethylhexyl) trimellitate (TOTM) may be cited. These plasticizers may be used either singly or in the form of a mixture of two or more members. Among other plasticizers mentioned above, DOP and DnDP prove advantageous. The content of the plasticizer is preferably in the range of from 30 to 70 parts by weight, based on 100 parts by weight of polyvinyl chloride, though variable with the kind of plasticizer and the bag to be used.

Other materials which are usable for the sheet material of the component bags 10 through 50 include polyolefins, namely the products obtained by polymerizing or copolymerizing such olefins or diolefins as ethylene, propylene, butadiene, and isoprene. As concrete examples of the material, polyethylene, poly-propylene, ethylene-vinyl acetate copolymer (EVA), polymer blends of EVA with various thermoplastic elastomers, and arbitrary combinations thereof may be cited. Besides, such polyesters as polyethylene terephthalate (PET), polybutylene terephthalate (PBT), and poly-1,4-cyclohexane dimethyl terephthalate (PCHT) and polyvinylidene chloride are also usable.

The thickness of the sheet material for constructing the component bags 10 through 50 is determined in consideration of such factors as permeability of sheet for such gases as oxygen gas and carbon dioxide gas and strength of sheet to withstand the impact of the centrifugation. Generally, the thickness of the sheet material for the blood collecting bag 10 is in the approximate range of from 0.2 to 1.0 mm, preferably from 0.3 to 0.5 mm, the thicknesses of the sheet materials for the blood plasma bag 20 and the chemical liquid storing bag 40 are each in the approximate range of from 0.2 to 0.7 mm, preferably from 0.3 to 0.5 mm, and the thickness of the sheet material for the buffy coat bag 30 is in the approximate range of from 0.2 to 1.0 mm, preferably from 0.3 to 0.5 mm. The sheet material for the blood platelet bag 50 advantageously has a relatively small thickness in the approximate range of from 0.1 to 0.6 mm, preferably from 0.2 to 0.45 mm, for the sake of improving the ability of the sheet to preserve blood platelets.

Though the inner volume of the blood collecting bag 10 is not particularly limited, it must be commensurate with the amount of blood to be collected. It is preferably in the approximate range of from 200 to 400 ml for the bags to be used in the territory of Japan and in the approximate range of from 350 to 600 ml for the bags to be used in foreign countries.

Though the inner volumes of the blood plasma bag 20, the chemical liquid storing bag 40, and the blood platelet bag 50 are not particularly limited, it is advantageous in the approximate range of from 100 to 400 ml, preferably from 150 to 300 ml, for the bags to be used in the territory of Japan and in the approximate range of from 150 to 600 ml, preferably from 200 to 450 ml for the bags to be used in foreign countries.

The inner volume of the buffy coat bag 30 is less than that of the blood collecting bag 10 and advantageously smaller than that of the blood plasma bag 20 and that of the chemical liquid storing bag 40. To be specific, the inner volume of the buffy coat bag 30 is advantageous in the approximate range of from 12 to 60%, preferably from 20 to 40%, of that of the blood collecting bag 10.

The materials which are usable for the construction of the tubes 15, 16, 18, 25, 35, 46, and 56 and the short tubes 170 and 340 in the multiple bags 1 and 2 include polyvinyl chloride, polyethylene, polypropylene, polyesters such as PET and PBT, ethylene-vinyl acetate copolymer, polyurethane, silicone, polyester elastomers, styrenebutadiene-styrene copolymer and other similar thermoplastic elastomers, for example. Among other materials enumerated above, polyvinyl chloride and materials using polyvinyl chloride as a main component thereof prove particularly advantageous. The reason for this advantage is that the tubes made of polyvinyl chloride possess enough flexibility and softness for permitting easy handling of themselves, allowing effective use of a clamp thereon, and ensuring highly desirable connection thereof to the relevant component bags. These tubes are not particularly discriminated on account of the kind and content of a plasticizer to be used therein.

Now, a preferred embodiment of this invention in the method for separation and transfer of blood components will be described below. This method of the invention for the separation and transfer of blood components is implemented by the use of the multiple bag of this invention described above. The use of the multiple bag 1 as an example is assumed in the description of the method for separation and transfer of blood components given below.

(A-1) The blood collecting needle 152 is plunged into the blood vessel to introduce blood into the blood storing part 13 of the blood collecting bag 10 having an anticoagulant (63 ml of the CPD liquid, for example) preparatorily contained therein. The amount of the blood to be collected is in the range of from 200 to 400 ml or from 450 to 600 ml. The following description assumes collection of 600 ml of the blood.

(A-2) After the collection of blood in the blood collecting bag 10 is completed, a midway part of the tube 15 is sealed by melting as with a tube sealer, for example, and the sealed part is cut to separate and remove the part of the tube on the blood collecting needle 152 side.

(A-3) Then, the blood collecting bag 10, blood plasma bag 20, buffy coat bag 30, and chemical liquid storing bag 40 are bundled, placed in a centrifugation cup of a centrifugal separator with the bottom parts of the bags kept on the lower side, and subjected to centrifugation. A centrifugal separator produced by Hitachi Koki Co., Ltd. and marketed under product code of "CR7B3," for example, may be used for the purpose of this centrifugation. The conditions for the centrifugation are 3500 to 4500 rpm (3600 to 5500 G) in speed and about 6.5 to 8 minutes in duration.

In consequence of the centrifugation so carried out, the blood in the blood collecting part 13 is separated into substantially three layers, i.e. an upper layer of blood plasma, an intermediate layer of buffy coat, and a lower layer of erythrocyte (not shown). The components which form these separated layer are variable with the conditions of centrifugation.

(A-4) After the centrifugation, the multiple bag 1 is gently taken out of the centrifugation cup and the blood collecting bag 10 is set in place on the separation stand (not shown).

The separation stand may be designed for manual operation or operation with a motor. The manual separation stand is in a simple construction comprising a pair of pressing plates and a lever attached to one of the pressing plates and operated by a procedure of setting the blood collecting bag 10 between the two pressing plates, manipulating the lever thereby rotating one of the pressing plates around one side thereof as the center toward the other pressing plate, and consequently enabling the blood collecting bag 10 to be nipped and compressed therebetween.

Alternatively, an automatic separation device which is provided with a bag compressing device (an electrically operated separation stand), a sensor for detecting the boundaries of separated components, and control means (microcomputer) may be used.

(A-5) The midway part of the tube 16 is pinched as with a clamp for complete closure of the inner hole thereof, the inner flow path of the sealing member 17 is opened as described above, and the separation stand is actuated to compress the blood collecting bag 10 gradually. As a result, the blood plasma (first blood component) of the upper layer is discharged through the opened sealing member 17 and transferred to the blood plasma storing part 23 of the blood plasma bag 20 through the medium of the tube 18, branching connector 19, and tube 25 to be recovered. This transfer of the blood plasma is stopped after the boundary between the buffy coat layer and the blood plasma layer has reached the upper part of the blood storing part 13 inside the blood collecting bag 10. The amount of the blood plasma (PPP) which is thus recovered is generally in the approximate range of from 250 to 280 ml.

(A-6) The midway part of the tube 18 is nipped as with a clamp for complete closure of the inner hole thereof, the tube 16 is relieved of the closure and simultaneously the inner flow path of the sealing member 34 is opened as described above, and the separation stand is actuated to compress the blood collecting bag 10 further. As a result, the buffy coat (second blood component) of the intermediate layer is transferred to the buffy coat storing part 33 of the buffy coat bag 30 through the medium of the tube 16 and the opened sealing member 34 to be recovered. The transfer of the buffy coat is stopped after the boundary between the erythrocyte layer and the buffy coat layer has reached the upper part of the blood storing part 13 inside the blood collecting bag 10. The amount of the buffy coat (BS) so recovered in this case is generally in the approximate range of from 50 to 60 ml. The erythrocyte (CRC) of the lower layer which is the third blood component now remain in the blood storing part 13 of the blood collecting bag 10.

(A-7) The midway part of the tube 16 is again pinched as with a clamp for perfect closure of the inner hole thereof, the tube 18 is relieved of the closure, the inner flow path of the sealing member 45 is opened as described above, and the chemical liquid storing bag 40 is compressed or raised to a level higher than the blood collecting bag 10. As a result, the erythrocyte preserving liquid 47 (100 ml of the S.A.G.M. liquid, for example) stored in the chemical liquid storing part 43 of the chemical liquid storing bag 40 is discharged through the opened sealing member 45 and transferred to the blood storing part 13 of the blood collecting bag 10 through the medium of the tube 46, branching connector 19, tube 18, and opened sealing member 17.

(A-8) After the whole amount of the erythrocyte preserving liquid 47 has been transferred into the blood collecting bag 10, the midway parts of the tubes 16, 18, and 25 are severally sealed by melting and the sealed parts are cut to separate the blood collecting bag 10, blood plasma bag 20, buffy coat bag 30, and chemical liquid storing bag 40 asunder. As a result, the blood collecting bag 10, the blood plasma bag 20, and the buffy coat bag 30 respectively storing erythrocyte, blood plasma, and buffy coat each in a tightly sealed state are obtained.

(A-9) A buffy coat pooling bag (not shown) is prepared separately and a tube connected to this bag is sterilely connected to the tube 16 which is connected to the buffy coat bag 30 by the use of a tube connecting device, for example. EP-B-0,044,204, for example, provides information on the tube connecting device and the method for connecting tubes. After the tubes have been connected, the contents of the buffy coat bag 30 are transferred into the buffy coat pooling bag by virtue of drop (difference in level).

The buffer coat enough for three to six recipients is stored in the buffy coat pooling bag by repeating the sterile connection of the buffy coat bag 30 containing the buffy coat from another donor to the aforementioned buffy coat pooling bag and the transfer of the buffy coat in the buffy coat bag 30 to the buffy coat pooling bag 3 to 5 times, for example, by following the procedure described above.

Either before or after the transfer of the contents of the buffy coat bag 30 to the buffy coat pooling bag for recovery therein, 30 to 60 ml, for example, of the blood platelet preserving liquid is injected through the medium of a sterilely connected tube into the buffy coat bag 30 to clean the interior thereof and the buffy coat now containing the blood platelet preserving liquid is recovered in the buffer coat pooling bag.

(A-10) The midway part of the tube interconnecting the buffy coat pooling bag and the buffy coat bag 30 is sealed as by melting and the sealed part is cut to separate and remove the buffy coat bag 30. Another tube connected to the buffy coat pooling bag is sterilely connected to a tube connected to a separately prepared blood platelet bag (not shown) in the same manner as described above.

Then, the buffy coat pooling bag and the blood platelet bag which are interconnected with a tube are set in place in the centrifugation cup of the centrifugal separator in the same manner as described above and then subjected to centrifugation. The conditions for this centrifugation are 750 to 1100 rpm (170 to 350 G) in speed and about 5 to 10 minutes in duration, for example.

In consequence of the centrifugation so carried out, the buffy coat in the buffy coat pooling bag is separated into two layers, i.e. an upper layer of blood platelets (concentrated blood platelets) and a lower layer containing leukocyte and erythrocyte richly (not shown).

(A-11) After the centrifugation, the double bag comprising the buffy coat pooling bag and the blood platelet bag is gently taken out of the centrifugation cup and the buffy coat pooling bag is set in place on the same separation stand as mentioned above and gradually compressed. As a result, the blood platelets of the upper layer are transferred into the blood platelet bag through the medium of the tube and the lower layer richly containing leukocyte and erythrocyte remains in the buffy coat pooling bag. This transfer of blood platelets is stopped after the boundary between the lower layer and the blood platelet layer has reached the upper end part of the buffy coat pooling bag.

(A-12) After the blood platelets in the buffy coat pooling bag have been substantially wholly transferred into the blood platelet bag, the midway part of the tube interconnecting these two bags is sealed as by melting and the sealed part is cut to separate the buffy coat pooling bag and the blood platelet bag from each other. As a result, the blood platelet bag which contains blood platelets in a tightly sealed state is obtained. Incidentally, the blood platelet bag is subjected to shaken preservation at normal room temperature, for example.

Now, the method for separation and transfer of blood components by the use of the multiple bag 2 described above will be explained below.

(B-1) and (B-2) The procedure of the steps (A-1) and (A-2) mentioned above is followed.

(B-3) The blood collecting bag 10, blood plasma bag 20, buffy coat bag 30, and blood platelet bag 50 are bundled and subjected to centrifugation in the same manner as in the step (A-4) mentioned above.

(B-4) The procedure of the step (A-4) mentioned above is repeated.

(B-5) The inner flow path of the sealing member 34 is opened in the as manner as described above and the separation stand is operated to compress the blood collecting bag 10 and introduce the blood plasma of the upper layer in the blood collecting bag 10 into the buffy coat storing part 33 of the buffy coat bag 30 through the medium of the tube 16, branching connector 19, tube 35, and opened sealing member 34. The amount of the blood plasma so introduced must be enough to induce floatation of blood platelets during the recovery of concentrated blood platelets from the buffy coat. Preferably, it is in the approximate range of from 10 to 60 ml.

Optionally, this step may be omitted.

(B-6) The midway part of the tube 16 is closed as with a clamp, the inner flow path of the sealing member 17 is opened as described above, and the separation stand is operated to compress the blood collecting bag 10. As a result, the blood plasma of the upper layer is discharged through the opened sealing member 17, and transferred via the tube 18 into the blood plasma storing part 23 of the blood plasma bag 20 to be recovered therein. This transfer of the blood plasma is stopped after the boundary between the buffy coat layer and the blood plasma layer has reached the upper part of the blood storing part 13.

(B-7) The midway part of the tube 18 is closed as with a clamp, the tube 16 is relieved of closure, and the separation stand is operated to compress the blood collecting bag 10 further. As a result, the buffy coat of the intermediate layer is transferred into the buffy coat storing part 33 of the buffy coat bag 30 through the medium of the tube, branching connector 19, tube 35, and opened sealing member 34 to be recovered therein. This transfer of the buffy coat is stopped after the boundary between the erythrocyte layer and the buffy coat layer inside the blood collecting bag 10 has reached the upper part of the blood storing part 13.

(B-8) The midway part of the tube 16 is again closed as with a clamp, the inner flow path of the sealing member 55 is opened as described above, and the blood platelet bag 50 is either compressed or raised to a level higher than the blood collecting bag 10 so as to allow about 5 ml of the erythrocyte preserving liquid (S.A.G.M. liquid, for example) stored in the blood platelet storing part 53 of the blood platelet bag 50 to be discharged through the opened sealing member 55 and passed through the tube 56, branching connector 19, tube 35, and opened sealing member 34 to clean these flow paths.

(B-9) The midway part of the tube 35 is closed as with a clamp, the tube 16 is relieved of closure, and the discharge of the erythrocyte preserving liquid 47 from the blood platelet bag 50 is continued. As a result, the erythrocyte preserving liquid 47 is transferred to the blood storing part 13 of the blood collecting bag 10 through the medium of the opened sealing member 55, tube 56, branching connector 19, tube 16, and opened sealing member 17.

(B-10) After the whole amount of the erythrocyte preserving liquid 47 has been transferred into the blood collecting bag 10, the midway parts of the tubes 16 and 18 are severally sealed by melting, and the sealed parts are cut to separate the multiple bag comprising the blood collecting bag 10, blood plasma bag 20, buffy coat bag 30, and blood platelet bag 50 asunder. As a result, the blood collecting bag 10 containing erythrocyte in a tightly sealed state and the blood plasma bag 20 containing blood plasma in a tightly sealed state are obtained.

(B-11) The buffy coat bag 30 and the blood platelet bag 50 which are interconnected with the tube 35, branching connector 19, and tube 56 are subjected to centrifugation in the same manner as in the step (A-10) mentioned above. As a result, the buffy coat in the buffy coat bag 30 is separated into substantially two layers, i.e. an upper layer of blood platelets (concentrated blood platelets) and a lower layer containing leukocyte and erythrocyte richly (not shown).

(B-12) After the centrifugation, the buffy coat bag 30 is gradually compressed in the same manner as in the step (A-10) described above. As a result, the blood platelets of the upper layer are transferred into the blood platelet storing part 53 of the blood platelet bag 50 via the opened sealing member 34, tube 35, branching connector 19, tube 56, and opened sealing member 55 and recovered therein and the lower layer containing leukocyte and erythrocyte richly remains in the buffy coat bag 30. This transfer of blood platelets is stopped after the boundary between the lower layer and the blood platelet layer has reached the upper part of the buffy coat storing part 33.

(B-13) After the blood platelets in the buffy coat bag 30 have been substantially wholly transferred to the blood platelet bag 50, the midway parts of the tubes 35 and 56 are severally sealed by melting and the sealed parts are cut to separate the buffy coat bag 30 and the blood platelet bag 50. As a result, the blood platelet bag 50 containing blood platelets in a tightly closed state is obtained. Incidentally, the blood platelets 50 are subjected to shaken preservation at a normal room temperature.

The methods described above can prevent blood and its separated components from contamination by microorganic invasion because they implement the separation of the blood into blood plasma, erythrocyte, blood platelets, etc. and the storage of necessary blood components in relevant bags invariably in a sterile manner. As a natural consequence, blood products are manufactured with high yields and the blood products are excellent in quality.

Now, the multiple bags of this invention and the method for separation and transfer of blood components by the use thereof will be described more specifically below with reference to working examples.

Example 1

A quadruple bag 1 constructed as shown in Fig. 1 was manufactured. The conditions of the component bags thereof were as shown below.

(1) Blood collecting bag 10
    Material - Flexible polyvinyl chloride (plasticizer: DEHP)
    Sheet thickness - About 0.36 mm
    Inner volume of bag - About 500 ml
    Content - CPD liquid 70 ml
(2) Blood plasma bag 20
    Material - Flexible polyvinyl chloride (plasticizer: DEHP)
    Sheet thickness - 0.36 mm
    Inner volume of bag - About 400 ml
(3) Buffy coat bag 30
    Material - Flexible polyvinyl chloride (plasticizer: DEHP)
    Sheet thickness - 0.36 mm
    Inner volume of bag - About 150 ml
(4) Chemical liquid storing bag 40
    Material - Flexible polyvinyl chloride (plasticizer: DEHP)
    Sheet thickness - 0.36 mm
    Inner volume of bag - About 400 ml
    Content - S.A.G.M. liquid 100 ml
(5) Tubes 15, 16, 18, 25, and 46
    Material - Flexible polyvinyl chloride (plasticizer: DEHP)
    Inside diameter - 3.0 mm
(6) Sealing members 17 and 45
    Construction - As shown in Fig. 3
    Short tube material - Flexible polyvinyl chloride (plasticizer: DEHP)
    Material for tubular part - Polycarbonate
(7) Sealing member 34
    Construction - As shown in Fig. 4
    Short tube material - Flexible polyvinyl chloride (plasticizer: DEHP)
    Material for tubular part - Polycarbonate
(8) Branching connector 19
    Material - Flexible polyvinyl chloride (plasticizer: DEHP)

Example 2

A quadruple bag 2 constructed as shown in Fig. 2 was manufactured. The conditions of the component bags thereof were as shown below.

(1) Blood collecting bag 10
    Same as in Example 1.
(2) Blood plasma bag 20
    Same as in Example 1.
(3) Buffy coat bag 30
    Same as in Example 1.
(4) Blood platelet bag 50
    Material - Flexible polyvinyl chloride (plasticizer: Mixture of DnDP and DEHP)
    Sheet thickness - 0.34 mm
    Inner volume of bag - About 400 ml
    Content of bag - S.A.G.M. liquid 100 ml
(5) Tubes 15, 16, 18, 35, and 56
    Same as in Example 1.

(6) Sealing members 17 and 55
    Same as in Example 1.
(7) Sealing member 34
    Same as in Example 1.
(8) Branching connector 19
    Same as in Example 1.


Control

A quadruple bag 3 constructed as shown in Fig. 5 was manufactured. The conditions of the bags 10, 20, 30, and 40, the tubes 61, 62, and 63, the sealing members 17 and 45, and the branching connectors 19 and 64 were the same as in Example 1.


1. Operations of separation and transfer of blood components

The multiple bag 1 of Example 1 was sterilized in an autoclave. The steps (A-1) through (A-12) mentioned above were carried out by the use of this multiple bag. Consequently, the blood collecting bag 10 containing concentrated erythrocyte (CRC), the blood plasma bag 20 containing blood plasma (PPP), and a pool of concentrated blood platelets (PC) enough for four recipients were obtained.

The multiple bag 2 of Example 2 was sterilized in an autoclave. The steps (B-1) through (B-13) mentioned above were carried out by the use of this multiple bag 2. Consequently, the blood collecting bag 10 containing concentrated erythrocyte (CRC), the blood plasma bag 20 containing blood plasma (PPP), and the blood platelet bag 50 containing concentrated blood platelets (PC) were obtained.

The multiple bag 3 of Control was sterilized in an autoclave. The steps (A-1) through (A-12) mentioned above were carried out by the use of this multiple bag. Consequently, the blood collecting bag 10 containing concentrated erythrocyte (CRC), the blood plasma bag 20 containing blood plasma (PPP), and the blood platelet bag containing concentrated blood platelets (PC) were obtained.

In each of the procedures of Examples 1 and 2 and Control, the conditions for the first centrifugation were 4140 rpm (5000 G) and 7 minutes 30 seconds and those for the second centrifugation were 900 rpm (240 G) and 10 minutes.


2. Test

The results of Examples 1 and 2 and Control were severally tested for the following items.
. Amount of blood collected
. Hematocrit of collected blood (%)
  Device - Automatic blood cell counter (produced by Toa Iyodenshi K.K. and marketed under trademark designation of Sysmex NE-6000)
. Amount of concentrated erythrocyte recovered
. Hematocrit (%) of concentrated erythrocyte
  Device - The automatic blood cell counter mentioned above
. Ratio of removal of leukocyte (%) from concentrated erythrocyte
  Method for determination - The count of leukocyte in a collected blood and the count of leukocyte in a concentrated erythrocyte were found by the use of the automatic blood cell counter. Based on these counts, the total amounts of leukocyte in the whole amounts of the blood were calculated. The ratio of the latter total number to the former total was calculated and reported in percentage as the ratio of residual leukocyte. Then, the ratio of residual white blood cells was subtracted from 100 and the difference was reported as the ratio of removal of leukocyte.
. Ratio of recovery of blood platelets (%)
  Method of determination - The count of blood platelets in a collected blood and the count of blood platelets in concentrated erythrocyte were found by the use of the automatic blood cell counter. Based on these counts, the total amounts of blood platelets in the whole amounts of the blood were calculated. The ratio of the latter total to the former total was calculated and reported in percentage as the ratio of recovery of blood platelets.
The results of the test for the items mentioned above are shown in Table 1 below.

Table 1

Average ± SD

| | Collected blood | | Concentrated erythrocyte | | Ratio of removal of leukocyte (%) | Ratio of recovery of blood platelets (%) |
|---|---|---|---|---|---|---|
| | Amount collected (ml) | Hematocrit (%) | Amount collected (ml) | Hematocrit (%) | | |
| Example 1 | 514.5 ± 6.0 | 37.4 ± 4.3 | 261.2 ± 20.7 | 53.0 ± 2.9 | 79.8 ± 4.0 | 79.1 ± 5.5 |
| Example 2 | 516.5 ± 5.4 | 40.1 ± 1.6 | 276.9 ± 15.1 | 54.5 ± 6.2 | 80.3 ± 4.5 | 80.9 ± 4.1 |
| Control | 514.9 ± 4.1 | 39.5 ± 3.2 | 268.9 ± 17.1 | 53.5 ± 4.7 | 65.3 ± 9.1 | 71.2 ± 9.8 |

(n = 12, providing the ratio of recovery of blood platelets in Example 1 was n = 3 because the blood platelets were pooled in amount for 4 recipients)

EP 0 686 403 A1

Table 1 clearly indicates that in both Examples 1 and 2, the ratio of removal of leukocyte and the ratio of recovery of blood platelets from the concentrated erythrocyte were high.

In contrast, in Control, the ratio of removal of leukocyte and the ratio of recovery of blood platelets from the concentrated erythrocyte were both lower than those of Examples 1 and 2.

The low ratio of removal of leukocyte in Control may be logically explained by a postulate that during the discharge and transfer of buffy coat from the blood collecting bag 10, leukocyte stagnate and accumulate inside the sealing member 17 in the upper part of the blood collecting bag while buffy coat is passing through the sealing member 17 and the accumulated leukocyte are suffered to mingle with erythrocyte preserving liquid while this liquid is in the process of transfer into the blood collecting bag 10 and are consequently returned into the blood collecting bag.

While there have been described the quadruple bag and the method for separation of transfer of blood components by the use of this bag, this invention does not need to be limited thereto. The quadruple bags 1 and 2 mentioned above may be dispossessed of the chemical liquid storing bag 40 and the tube 46 or of the blood platelet bag 50 and the tube 56 and consequently caused to form a triple bag. Otherwise, they may be additionally provided with some other bag (to be exclusively used for storing erythrocyte, for example) and consequently caused to form a five-member bag.

This invention finds utility not only in the separation and transfer of the blood components mentioned above (particularly for the manufacture of blood products from whole blood) but also in the concentration of marrow fluid (removal of peripheral blood), cleansing of erythrocyte, etc.

## Claims

1. A multiple bag (1,2) comprising a first (10) bag for storing blood, a second bag (20) for storing a first blood component formed mainly of blood plasma separated from said blood, a third bag (30) for storing a second blood component containing leukocyte separated from said blood, a first tube (18) for interconnecting said first bag (10) and said second bag (20) in such a manner as to establish communication between the interiors of the bags, and a second tube (16) for interconnecting said first bag (10) and said third bag (30) in such a manner as to establish communication between the interiors of said bags, characterized in that a sealing member (17) capable of being broken so as to open an inner flow path is provided in the neighborhood of the part for connecting said second tube (16) to said third bag (30) and, by opening said inner flow path of said sealing member (17), said first bag (10) and said third bag (30) are enabled to communicate with each other through the medium of said second tube (16).

2. A multiple bag according to claim 1, wherein said sealing member (17) is so constructed that a broken fragment separated therefrom by breakage is drawn in to a position at which it is incapable of interfering with the flow of said blood component therethrough.

3. A multiple bag according to claim 1 or claim 2, wherein said first bag (10) is used as a bag for receiving and storing a third blood component containing erythrocyte separated from said blood.

4. A multiple bag according to any of claims 1 through 3, which further comprises a fourth bag (40,50) containing a chemical liquid.

5. A multiple bag according to claim 4, which further comprises a third tube (46,56) branching from said first tube (16) and connected to said fourth bag (40) so as to communicate with the interior of said fourth bag (40).

6. A multiple bag according to claim 4, which further comprises a third tube (46,56) branching from said second tube (16) and connected to said fourth bag (40,50) so as to communicate with the interior of said fourth bag (40,50).

7. A multiple bag according to claim, wherein said fourth bag (40,50) is used as a bag for receiving and storing a fourth blood component containing platelets separated further from said second blood component.

8. A method for separating and transferring blood components by the use of a multiple bag (1,2) set forth in any of claims 1 through 7, characterized by comprising a step of subjecting the blood stored in said first bag (10) to centrifugation thereby separating said blood into at least three components, i.e. an upper layer formed of said first blood component, an intermediate layer formed of said second blood component, and a lower layer formed of said third blood component containing erythrocyte, a step of transferring said first blood component to said second bag (20) through the medium of said first tube (18), and a step of transferring said second blood component to said third bag (30) through the medium of said second tube (16) and said sealing member (17) in an opened state.

9. A method for separating and transferring blood components by the use of a multiple bag set forth in claim 5, characterized by comprising a step of subjecting the blood stored in said first bag (10) to centrifugation thereby separating said blood into at least three components, i.e. an upper layer formed of said first blood component, an intermediate layer formed of said second blood component, and a lower layer formed of said third blood component containing erythrocyte, a step of transferring said first blood component to said second bag (20) through the medium of the said tube (18), a step of transferring said second blood component to said third bag (30) through the medium of said second tube (16) and said sealing member (17) in an opened state, and a step of transferring the chemical liquid in said fourth bag to said first bag (10) through the medium of said third tube (25) and said first tube (18).

10. A method for separating and transferring blood components by the use of a multiple bag set forth in claim 6 or claim 7, characterized by comprising a step of subjecting the blood stored in said first bag (10) to centrifugation thereby separating said blood into at least three components, i.e. an upper layer formed of said first blood component, an intermediate layer formed of said second blood component, and a lower layer formed of said third blood component containing erythrocyte, a step of transferring said first blood component to said second bag (20) through the medium of said first tube (18), a step of transferring said second blood component to said third bag (30) through the medium of said second tube (16), and said sealing member (17) in an opened state, and a step of transferring the chemical liquid in said fourth bag (40,50) to said first bag (10) through the medium of said third tube (46,56) and said second tube (16).

11. A method for separating and transferring blood components by the use of a multiple bag set forth in claim 6 or claim 7, characterized by comprising a step of subjecting the blood stored in said first bag (10) to centrifugation thereby separating said blood into at least three components, i.e. an upper layer formed of said first blood component, an intermediate layer formed of said second blood component, and a lower layer formed of said third blood component containing erythrocyte, a step of transferring said first blood component to said second bag (20) through the medium of said first tube (18), a step of transferring said second blood component to said third bag (30) through the medium of said second tube (16) and said sealing member (17) in an opened state, a step of transferring the chemical liquid in said fourth bag (40,50) to said first bag (10) mentioned above through the medium of said third tube (18) and said second tube (16), a step of subjecting said second blood component stored in said third bag (30) thereby obtaining an upper layer mainly containing platelets, and a step of transferring the platelets of said upper layer to said fourth bag (40,50) through the medium of said second tube (16) and said third tube (46,56).

# FIG.I

151 152 153

15

46

1

14 16 17 18 19 25 24 44 45

344 34 30 20 40

331 331

13 33

12 32 23 47 43

11 31 22 42 41

21

EP 0 686 403 A1

# FIG.2

# FIG.3

# FIG.4

EP 0 686 403 A1

FIG.5

| | **European Patent Office** | **EUROPEAN SEARCH REPORT** | Application Number<br>**EP 95 10 8828** |

## DOCUMENTS CONSIDERED TO BE RELEVANT

| Category | Citation of document with indication, where appropriate,<br>of relevant passages | Relevant<br>to claim | CLASSIFICATION OF THE<br>APPLICATION (Int.Cl.6) |
|---|---|---|---|
| X | EP-A-0 536 594 (E NELSON)<br>* column 8, line 37 - column 9, line 34 *<br>* figures 1,2 *<br>--- | 1 | A61M1/02 |
| A | EP-A-0 600 804 (TERUMO KABUSHIKI KAISHA)<br>* page 7, line 37 - line 54 *<br>* page 10, line 42 - page 11, line 6 *<br>* page 16, line 30 - line 54 *<br>* figures 1,7 *<br>--- | 1,2,8-11 | |
| A | EP-A-0 573 405 (OEKO WIEN)<br>* the whole document *<br>--- | 1 | |
| P,X | WO-A-94 28996 (BAXTER INT INC)<br>* page 9, line 15 - page 10, line 4 *<br>* page 11, line 2 - line 28 *<br>* figure 1 *<br>----- | 1 | |
| | | | TECHNICAL FIELDS<br>SEARCHED (Int.Cl.6)<br><br>A61M |

The present search report has been drawn up for all claims

| Place of search | Date of completion of the search | Examiner |
|---|---|---|
| THE HAGUE | 12 September 1995 | Vereecke, A |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

...........................................................................
& : member of the same patent family, corresponding
   document

EPO FORM 1503 03.82 (P04C01)